(19) Europäisches Patentamt
European Patent Office
Office européen des brevets

(11) **EP 3 375 355 A1**

(12) **EUROPÄISCHE PATENTANMELDUNG**

(43) Veröffentlichungstag:
**19.09.2018 Patentblatt 2018/38**

(51) Int Cl.:
***A61B 5/00*** *(2006.01)*

(21) Anmeldenummer: **17161538.8**

(22) Anmeldetag: **17.03.2017**

| | |
|---|---|
| (84) Benannte Vertragsstaaten:<br>**AL AT BE BG CH CY CZ DE DK EE ES FI FR GB GR HR HU IE IS IT LI LT LU LV MC MK MT NL NO PL PT RO RS SE SI SK SM TR**<br>Benannte Erstreckungsstaaten:<br>**BA ME**<br>Benannte Validierungsstaaten:<br>**MA MD** | (71) Anmelder: **bluepoint medical GmbH & Co. KG**<br>**23923 Selmsdorf (DE)**<br><br>(72) Erfinder: **LINDNER, Bernd**<br>**23167 Stockelsdorf (DE)**<br><br>(74) Vertreter: **Seemann & Partner Patentanwälte mbB**<br>**Raboisen 6**<br>**20095 Hamburg (DE)** |

(54) **ANORDNUNG UND VERFAHREN ZUR PULSOXYMETRIE SOWIE VERWENDUNG**

(57)     Die Erfindung betrifft eine Anordnung zur Puls-oxymetrie, umfassend wenigstens einen Sensor (10, 30), der einen Sender (12, 32) mit einer oder mehreren Lichtquellen und einen Empfänger (14, 34) für von dem Sender (12, 32) durch ein Gewebe gestrahltes Licht aufweist, sowie ein Verfahren zur Pulsoxymetrie und eine Verwen-dung.

Erfindungsgemäß sind in der Anordnung ein oder mehrere beidseitig adhäsive flächige Hydrogel-Haftkissen (20, 22, 40, 42, 44, 46, 50, 54, 58) umfasst, das oder die zwischen dem Sender (12, 32) und einer Oberfläche des Gewebes und/oder zwischen dem Empfänger (14, 34) und einer Oberfläche des Gewebes anzuordnen oder angeordnet ist oder sind, wobei im zusammengesetzten Zustand der Anordnung das oder die Hydrogel-Haftkissen (20, 22, 40, 42, 44, 46, 50, 54, 58) eine Anhaftung des Senders (12, 32) und/oder des Empfängers (14, 34) an der Oberfläche des Gewebes vermittelt oder vermitteln und für einen oder mehrere für die Pulsoxymetrie verwendeten Anteile eines Wellenlängenbereichs des durch das Gewebe gestrahlten Lichts transparent ist oder sind.

Fig. 2

**Beschreibung**

Die Erfindung betrifft eine Anordnung und ein Verfahren zur Pulsoxymetrie sowie eine Verwendung.

Die Pulsoxymetrie ist ein Verfahren zur nichtinvasiven photometrischen Ermittlung der arteriellen Sauerstoffsättigung über die Messung der Lichtabsorption bei Durchleuchtung eines blutdurchströmten Gewebes oder bei Streuung des Lichts in dem Gewebe. Typische Anwendung findet die Messung an leicht zugänglichen Extremitäten, wie Finger, Zehen, Ohrläppchen oder der Ferse oder am Handgelenk bei Säuglingen. Die so ermittelte Sauerstoffsättigung wird $S_pO_2$ bezeichnet. Diese berechnet sich als

$$S_pO_2 = \frac{HbO_2}{HbO_2 + Hb},$$

wobei $HbO_2$ die Konzentration des mit $O_2$ beladenen Hämoglobins, des oxygenierten Hämoglobins bzw. Oxyhämoglobins, und $Hb$ die Konzentration des desoxygenierten Hämoglobins, dessen Transportplätze für $O_2$ noch frei sind, bezeichnen.

Entsprechende Sensoren haben auf einer Seite beispielsweise eine oder zwei in einem definierten Infrarotbereich und gegebenenfalls im sichtbaren Bereich leuchtende Lichtquellen, auf der anderen Seite einen Fotosensor. Das Licht dringt in das Gewebe ein und wird auf dem Weg zum Sensor durch das Gewebe, die Blutgefäße und das Blut teilweise absorbiert. Oxygeniertes und desoxygeniertes Hämoglobin haben unterschiedliche Farben und damit auch ein unterschiedliches Absorptionsverhalten. Gemessen werden üblicherweise zwei bis drei Werte, nämlich die Absorption des Lichts bei unterschiedlichen Wellenlängen, zumeist im Bereich von 660 nm bis 940 nm.

Durch den Herzschlag pulsieren die arteriellen Blutgefäße. Damit ändert sich der Weg des Lichts durch das arterielle Blut. Es lässt sich bei jedem Herzschlag für jede Wellenlänge das Verhältnis von maximalem Wert der Lichtintensität zum minimalen Wert bilden. Wenn die Werte in Beziehung zueinander gesetzt werden, so ist das Ergebnis unabhängig von der Lichtabsorption des umliegenden Gewebes und nur bestellt durch das Verhältnis von oxygeniertem zu desoxygeniertem Hämoglobin im arteriellen Blut. In vielen Fällen wird durch Vergleich mit einer Referenztabelle der prozentuale Anteil an oxygeniertem Hämoglobin ermittelt.

Die Pulsoxymetrie wird im Rettungsdienst, auf Intensivstationen, in der Neonatalmedizin, in der Anästhesie und in der Schlafforschung routinemäßig eingesetzt und ist Teil des Standardmonitorings von Patienten. Weitere Anwendungen findet die Pulsoxymetrie bei belastenden Tätigkeiten wie dem Sportfliegen oder dem Bergsteigen. Kurzzeit-Pulsoxymetriesensoren werden in vielen Fällen als wiederverwendbare Clips eingesetzt, die gegebenenfalls die Auswertung über Elektronik und Anzeige bereits integriert haben. Diese werden beispielsweise auf einen Finger aufgesetzt und können problemlos wieder abgenommen werden. Im Langzeitmonitoring werden die Sensoren an der Messstelle auf die Haut aufgelegt und mittels Verbandsmaterial befestigt. Hierbei wird die Luftzufuhr zu den betroffenen abgedeckten Hautstellen verringert und diese beginnen zu schwitzen, sodass sich ein Flüssigkeitsfilm zwischen der Hautoberfläche und dem Sensor bilden kann. Ferner soll durch eine enge Wicklung des Verbandsmaterials verhindert werden, dass die Sensoren verrutschen, was bei längerer Auflage zu Druckstellen führt. Aus diesem Grund ist es notwendig, alle paar Stunden die Sensorposition und das Verbandsmaterial zu wechseln. Zusätzlich erhöht der Flüssigkeitsfilm die Gefahr, dass der Sensor durch Bewegungen verrutschen kann, sodass die Messung verfälscht wird bzw. es zu gehäuften Fehlalarmen kommt.

Demgegenüber liegt der vorliegenden Erfindung die Aufgabe zugrunde, bei Pulsoxymetriemessungen die Messgenauigkeit und Langzeitanwendbarkeit zu verbessern.

Diese Aufgabe wird durch eine Anordnung zur Pulsoxymetrie, umfassend wenigstens einen Sensor, der einen Sender mit einer oder mehreren Lichtquellen und einen Empfänger für von dem Sender durch ein Gewebe gestrahltes Licht aufweist, gelöst, die dadurch weitergebildet ist, dass ein oder mehrere beidseitig adhäsive flächige Hydrogel-Haftkissen umfasst sind, das oder die zwischen dem Sender und einer Oberfläche des Gewebes und/oder zwischen dem Empfänger und einer Oberfläche des Gewebes anzuordnen oder angeordnet ist oder sind, wobei im zusammengesetzten Zustand der Anordnung das oder die Hydrogel-Haftkissen eine Anhaftung des Senders und/oder des Empfängers an der Oberfläche des Gewebes vermittelt oder vermitteln und für einen oder mehrere für die Pulsoxymetrie verwendeten Anteile eines Wellenlängenbereichs des durch das Gewebe gestrahlten Lichts transparent ist oder sind.

Die Erfindung beruht auf dem Grundgedanken, dass Hydrogel-Haftkissen, die als Elektroden bislang verwendet worden sind, auch für die Pulsoxymetrie sehr vorteilhaft einsetzbar sind. Ein Hydrogel ist ein große Mengen von Wasser enthaltendes wasserunlösliches Polymer, dessen Molekülketten chemisch zu einem dreidimensionalen Netzwerk verknüpft sind. Durch eingebaute hydrophile Polymerkomponenten quellen sie in Wasser unter beträchtlicher Volumenzunahme und halten das Wasser nach der Aufnahme fest.

Aufgrund des großen Wasseranteils von teilweise bis zu 90 % oder mehr sind sie nicht nur leitfähig, was sie für Elektroden besonders geeignet macht, sondern auch durchsichtig für sichtbares und Infrarotlicht, eine Grundvoraussetzung für die Anwendung in der Pulsoxymetrie. Gleichzeitig ist Hydrogel hautverträglich und somit für eine Anwendung auf der Haut geeignet. Da es Wasser aufnimmt, nimmt es auch das Schwitzwasser aus der Haut auf, sodass sich die Hautverträglichkeit gegenüber einem direkten Auflegen der Sensoren auf die Haut verbessert.

Da die erfindungsgemäß eingesetzten Hydrogel-Haftkissen beidseitig adhäsiv sind, klebt sowohl der Sensor an dem Haftkissen als auch das Haftkissen an der Haut, sodass ein ungewolltes Verschieben des Sensors während der Messung unterbunden wird. Das verbessert die Langzeitstabilität der Messung. Es entfällt außerdem die Notwendigkeit, den Sensor unter Druck zu fixieren, so dass die Bildung von Druckstellen weitgehend vermieden werden kann.

Ein weiterer messtechnischer Vorteil ist, dass das Hydrogel-Haftkissen eine optische Dichte aufweist, die ähnlich zu derjenigen des zu untersuchenden Gewebes ist. Es finden somit keine starken Übergänge der optischen Dichte statt, wie dies bei den üblicherweise aufgelegten Sensoren der Fall ist, bei denen auch Luft zwischen dem Sensor und der Haut vorhanden ist. Die damit einhergehende Reflexion an der entsprechenden Luft/Gewebe-Phasengrenze entfällt, so dass die Einkopplung und die Auskopplung des zur Messung verwendeten Lichts in das zu untersuchende Gewebe verbessert werden.

Wenn vorteilhafterweise ein Pulsoxymetrie-Monitor umfasst ist, der dazu ausgebildet und mit dem Sensor derart verbunden ist, dass der Pulsoxymetrie-Monitor die Lichtquelle oder Lichtquellen steuert und Signale des Empfängers empfängt und auswertet, ist ein System zur Pulsoxymetrie verwirklicht, das deutlich länger als bisher am Patienten verbleiben kann und eine stabile Pulsoxymetriemessung ermöglicht. Hierbei kann es sich um einen Monitor handeln, der direkt mit dem Sensor in einer Einheit verbaut ist, oder über Kabel oder drahtlos mit dem Sensor verbunden ist.

Vorzugsweise ist oder sind das oder die Hydrogel-Haftkissen hautfreundlich und biokompatibel. Dies ist bei der geplanten Langzeitanwendung in der Pulsoxymetrie sehr wünschenswert. Ebenfalls bevorzugt ist es, wenn das oder die Hydrogel-Haftkissen rückstandslos von einer Hautoberfläche entfernbar ist oder sind. Diese Merkmale begünstigen den Einsatz von Hydrogel-Haftkissen in der Pulsoxymetrie besonders bei Menschen mit empfindlicher Haut, beispielsweise Neugeborenen. Es erleichtert auch die Nachsorge, da die Hautpartien, die mit einem Hydrogel-Haftkissen bedeckt waren, keiner weiteren Versorgung bedürfen.

Messtechnisch ist es von Vorteil, wenn das oder die Hydrogel-Haftkissen frei von Lufteinschlüssen oder anderen Einschlüssen ist oder sind. Lufteinschlüsse oder andere Einschlüsse im Hydrogel wirken als Streuzentren und vermindern die Intensität des in das Gewebe eingebrachten Lichts und somit die Genauigkeit der Messung.

Vorteilhafterweise weisen das oder die Hydrogel-Haftkissen einen Wassergehalt von mehr als 50%, insbesondere von mehr als 80%, auf. Entsprechende Hydrogele sind besonders geeignet, da sie eine hohe Transparenz für sichtbares und Infrarotlicht und eine besonders hohe Hautfreundlichkeit aufweisen.

Gängige Hydrogele sind sehr weich gegenüber Scherungen. Eine gewisse Verschiebung der Sensoren ist daher nicht ausgeschlossen. Um die Positionierungsstabilität der Anordnung zu verbessern, ist es vorteilhaft, wenn das oder die Hydrogel-Haftkissen einen stabilisierenden Rand, insbesondere aus einem Hydrocolloid, aufweisen. Ein solcher stabilisierender Rand dient dazu, das Verrutschen der Sensorteile auf dem Hydrogel-Haftkissen zu vermindern, indem eine stabile Randbegrenzung für das weiche Hydrogel geboten wird. Ein geeignetes Material hierfür ist ein Hydrocolloid, das ähnlich hautfreundliche Eigenschaften aufweist wie ein Hydrogel, jedoch einen geringeren Wasseranteil aufweist und aufgrund der Lichtstreuung an den Colloidtröpfchen auch nicht die entsprechende Transparenz. Hydrocolloide umfassen unter anderem Polysaccharide und Proteine, die in Wasser als Colloide in Lösung gehen und ein hohes Vermögen zur Gelbildung zeigen. Vorzugsweise weist der stabilisierende Rand eine Breite von wenigstens 1 mm auf. Mit der entsprechenden Breite ist die gewünschte Stützwirkung darstellbar. Der Sensor ist vorzugsweise als Y-Sensor oder in einer U-Konfiguration ausgebildet. Ein Y-Sensor weist ein Kabel mit Zuführung und Rückführung für die beiden Sensorteile, den Empfänger und den Sender, auf, welches sich in zwei Kabel aufgabelt, an deren Ende jeweils der Sender bzw. der Empfänger des Sensors sich befindet. Sender und Empfänger können dann an gegenüberliegenden Seiten beispielsweise eines Fingers oder eines Ohrläppchens angeordnet werden. Bei einer U-Konfiguration haben Sender und Empfänger einen gemeinsamen Kabelstrang und dieser wird zusammen mit Sender und Empfänger um die ausgewählte Extremität gebunden.

Die der Erfindung zugrunde liegende Aufgabe wird auch durch ein Verfahren zur Pulsoxymetrie gelöst, bei dem aus einem Sender mit einer oder mehreren Lichtquellen Licht durch ein mit Blut versorgtes Gewebe gestrahlt wird und von einem Empfänger aufgefangen wird, wobei aus einer Abschwächung des Lichts bei einer oder mehreren Wellenlängen auf eine Sättigung des in dem Gewebe enthaltenen Blutes mit Sauerstoff ermittelt wird, das dadurch weitergebildet ist, dass der Sender und/oder der Empfänger mittels eines oder mehrerer beidseitig adhäsiver flächiger Hydrogel-Haftkissen auf einer Oberfläche des Gewebes befestigt wird oder werden, wobei das Licht aus dem Sender vor Eintritt in das Gewebe und/oder nach Austritt aus dem Gewebe ein oder mehrere Hydrogel-Haftkissen durchstrahlt.

Die erfindungsgemäße Aufgabe hat die gleichen Merkmale, Eigenschaften und Vorteile wie die zuvor beschriebene erfindungsgemäße Anordnung.

Im Rahmen der vorliegenden Erfindung wird unter einem Durchstrahlen des Gewebes sowohl eine klassische Transmissionsmessung verstanden, bei der Licht an einer Seite in das Gewebe eintritt und an der anderen Seite austritt und gemessen wird, als auch eine Streumessung, bei der Licht für die Messung an einer Stelle in das Gewebe angestrahlt wird und seitlich versetzt gestreutes Licht aufgefangen wird. Auch in diesem Fall ist das Gewebe durchstrahlt worden.

Zur Vorbereitung der Messung wird oder werden vorzugsweise das oder die Hydrogel-Haftkissen zwischen einer Hautoberfäche oder einem Fingernagel einerseits und dem Sender und/oder Empfänger andererseits im Wesentlichen bündig angeordnet. Ferner werden, insbesondere bei Langzeitmonitoring, das oder die Hydrogel-Haftkissen und der Sender und der Empfänger am zu messenden Gewebe, insbesondere einem Finger, mit einem, insbesondere einseitig haftenden, Verbandsmaterial umwickelt.

Die der Erfindung zugrundeliegende Aufgabe wird ferner auch gelöst durch eine Verwendung von einem oder mehreren beidseitig adhäsiven flächigen und für Licht transparenten Hydrogel-Haftkissen zur Befestigung eines Senders und/oder eines Empfängers eines Pulsoxymetrie-Sensors auf einem mit Pulsoxymetrie zu überwachenden Gewebes in einem Lichtpfad des Sensors. Auch diese Verwendung bezieht sich auf die zuvor beschriebene erfindungsgemäße Anordnung und das zuvor beschriebene erfindungsgemäße Verfahren und teilt deren Merkmale, Vorteile und Eigenschaften.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:

Fig. 1   die wellenlängenabhängigen Auslöschungskoeffizienten verschiedener Zustände von Hämoglobin,

Fig. 2   zwei Ausführungsformen erfindungsgemäßer Anordnungen und

Fig. 3   Ausführungsformen erfindungsgemäß verwendbarer Hydrogel-Haftkissen.

In den Zeichnungen sind jeweils gleiche oder gleichartige Elemente und/oder Teile mit denselben Bezugsziffern versehen, so dass von einer erneuten Vorstellung jeweils abgesehen wird.

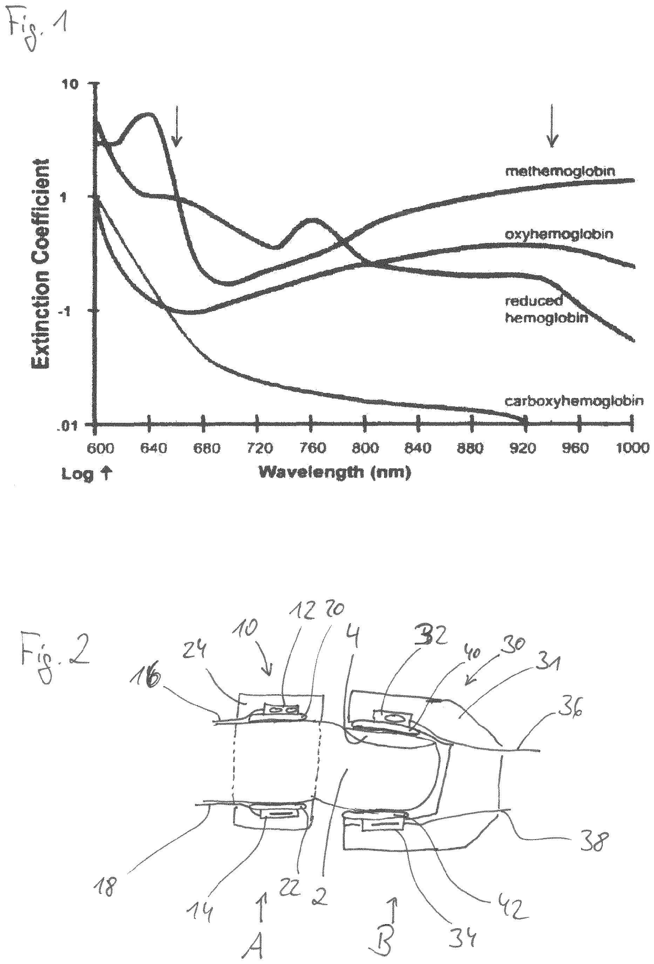

Figur 1 zeigt die wellenlängenabhängigen Auslöschungskoeffizienten für verschiedene Zustände von Hämoglobin, nämlich Methämoglobin, Oxyhämoglobin ($HbO_2$), reduziertes Hämoglobin (Hb) und Carboxyhämoglobin. Hauptbestandteile des Blutes sind Oxyhämoglobin und reduziertes Hämoglobin. Carboxyliertes Hämoglobin ist mit CO beladen und ist mit den wenigen Prozent im Blut vorhanden, während Methämoglobin entsteht, wenn das zweiwertige Eisen im Hämoglobin durch Anlagerung von Sauerstoff im Sinne einer Autooxidation zu dreiwertigem oxidiert wird. Methämoglobin kann keinen Sauerstoff mehr abgeben. Sein Anteil im Blut beträgt meist weniger als 1,5 %. Im Regelfall tragen daher Methämoglobin und Carboxyhämoglobin wenig zur Auslöschung von Licht in der Pulsoxymetrie bei.

Mit Pfeilen gekennzeichnet sind die Wellenlängenbereiche, in denen die Auslöschung des ausgesendeten Lichts gemessen wird. Dies erfolgt üblicherweise im Bereich um 660 nm bis um 940 nm. Im Infrarotbereich überwiegt die Auslöschung durch das Oxyhämoglobin diejenige durch das reduzierte Hämoglobin, während im roten Bereich die Verhältnisse umgekehrt sind. Es ist zu beachten, dass die Skala auf der vertikalen Achse logarithmisch ist. Wenn außerdem die Anteile von Methämoglobin und gegebenenfalls Carboxyhämoglobin ermittelt werden sollen, ist die Messung bei weiteren Wellenlängen notwendig.

In Figur 2 sind zwei verschiedene alternative Anordnungen (A, B) gezeigt, die jeweils an einem Finger 2, der auch einen Fingernagel 4 aufweist, angeordnet sind. In der Anordnung A umfasst der Sensor 10 einen Sender 12 mit mehreren Lichtquellen und einen Empfänger 14, die auf gegenüberliegenden Seiten des Fingers 2 voneinander angeordnet sind. Sowohl Sender 12 als auch Empfänger 14 sind jeweils mit einem Hydrogel-Haftkissen 20, 22 an dem Finger 2 angebracht. Licht von dem Sender 12 durchstrahlt den Finger 2 und gelangt zum Empfänger 14. Ebenfalls eingezeichnet sind Zuleitungen 16, 18 zu dem Sender 12 und dem Empfänger 14. Die Hydrogel-Haftkissen 20, 22 sorgen für eine klebende Verbindung zwischen Sender 12 und Empfänger 14 einerseits und dem Finger 2 andererseits. Die Hydrogel-Haftkissen 20, 22 können auch Schwitzwasser aufnehmen, so dass diese Anordnung länger als bisher am Patienten verbleiben kann, ohne dass sich der Sensor verschieben kann oder ein Wechsel des Verbandsmaterials 24 notwendig wird, welches zusätzlich um den Sensor 10 herum appliziert worden ist.

In der Konfiguration B der Anordnung ist der Sensor 30 als Clip 31 ausgebildet, der auf den Finger 2 klammernd gesteckt wird. In einem Arm des Clips 31 ist ein Sender 32 und im anderem Arm ein Empfänger 34 angeordnet, wobei, anders als bei herkömmlichen Pulsoxymetrie-Clips zusätzlich Hydrogel-Haftkissen 40, 42 zur Befestigung und zur Lichtleitung zwischen Sender 32 und Fingernagel 4 sowie zwischen Empfänger 34 und Oberfläche des Fingers 2 angeordnet werden, die den Clip und damit die Bestandteile des Sensors gegenüber dem Finger 2 stabilisieren. Auch im Falle des Sensors 30 sind zwei Zuleitungen 36, 38 zu dem Sender 22 und dem Empfänger 34 eingezeichnet, sodass eine Y-Anordnung realisiert ist. Ebenso gut könnte allerdings auch eine einzelne Zuleitung in den Clip eindringen und beispielsweise vom Sender 32 weiter zum Empfänger 34 verlaufen oder umgekehrt, und so zu einer U-Konfiguration führen.

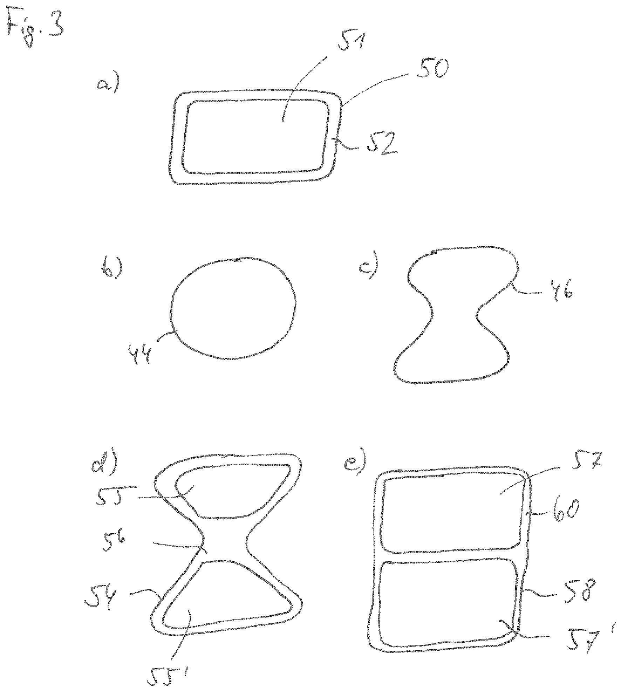

In Figur 3 sind verschiedene Ausführungsbeispiele erfindungsgemäß einsetzbarer Hydrogel-Haftkissen schematisch dargestellt. Das in Figur 3 a) dargestellte Hydrogel-Haftkissen 50 hat eine rechteckige Grundform, wobei die zentrale durchsichtige Hydrogel-Fläche 51 durch einen Hydrocolloid-Rand 52 umrandet ist, die dem Hydrogel-Haftkissen 50 eine erhöhte Stabilität im Randbereich verleiht.

In Figur 3 b) ist ein ovales Hydrogel-Haftkissen 44 ohne Rand dargestellt, dass Hydrogel-Haftkissen 46 gemäß Figur 3 c) hat im Vergleich dazu eine Umfangsform einer Sanduhr mit einer Einschnürung in der Mitte und lässt sich gut in einem Stück beispielsweise um einen Finger wickeln, sodass die beiden breiteren Flächen jeweils einen Ansatzpunkt für einen Sender und einen Empfänger eines Sensors eines Pulsoxymetrie-Sensors bieten.

Die Figuren 3 d) und 3 e) zeigen Hydrogel-Haftkissen 54 und 58, die jeweils ebenfalls zwei Flächen zum Befestigen jeweils eines Senders und eines Empfängers eines Pulsoxymetrie-Sensors bieten. Während das Hydrogel-Haftkissen 54 wiederum eine Sanduhrform aufweist, ist das Hydrogel Haftkissen 58 im Wesentlichen rechteckig. Beiden ist gemeinsam, dass sie jeweils einen Hydrocolloid-Rand 56 bzw. 60 aufweisen, der den Hydrogel-Haftkissen 54, 58 im Randbereich eine ausreichende Stabilität verleiht. Der Hydrocolloid-Rand 56 bzw. 60 trennt jeweils zwei Hydrogel-Flächen 55, 55' bzw. 57, 57' voneinander ab, so dass die Hydrogel-Haftkissen 54, 58 auch im zentralen Bereich eine ausreichende Stabilität aufweisen.

Aufgrund der hohen Wasseraufnahmefähigkeit und Hautverträglichkeit können solche Hydrogel-Haftkissen sehr lange, auch mehr als 24 Stunden, auf einer Stelle verbleiben, so dass eine ununterbrochene und gegen Verrutschen gesicherte Langzeitüberwachung per Pulsoxymetrie möglich ist.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

Bezugszeichenliste

| 2 | Finger |
|---|---|
| 4 | Fingernagel |
| 10 | Sensor |
| 12 | Sender |
| 14 | Empfänger |
| 16 | Zuleitung |
| 18 | Zuleitung |
| 20, 22 | Hydrogel-Haftkissen |
| 24 | Verbandsmaterial |
| 30 | Sensor |
| 31 | Clip |
| 32 | Sender |
| 34 | Empfänger |
| 36 | Zuleitung |
| 38 | Zuleitung |
| 40, 42 | Hydrogel-Haftkissen |
| 44, 46 | Hydrogel-Haftkissen |
| 50 | Hydrogel-Haftkissen |
| 51 | Hydrogel-Fläche |
| 52 | Hydrocolloid-Rand |
| 54 | Hydrogel-Haftkissen |
| 55, 55' | Hydrogel-Fläche |
| 56 | Hydrocolloid-Rand |
| 57, 57' | Hydrogel-Fläche |
| 58 | Hydrogel-Haftkissen |
| 60 | Hydrocolloid-Rand |

**Patentansprüche**

1. Anordnung zur Pulsoxymetrie, umfassend wenigstens einen Sensor (10, 30), der einen Sender (12, 32) mit einer oder mehreren Lichtquellen und einen Empfänger (14, 34) für von dem Sender (12, 32) durch ein Gewebe gestrahltes Licht aufweist, **dadurch gekennzeichnet, dass** ein oder mehrere beidseitig adhäsive flächige Hydrogel-Haftkissen (20, 22, 40, 42, 44, 46, 50, 54, 58) umfasst sind, das oder die zwischen dem Sender (12, 32) und einer Oberfläche des Gewebes und/oder zwischen dem Empfänger (14, 34) und einer Oberfläche des Gewebes anzuordnen oder angeordnet ist oder sind, wobei im zusammengesetzten Zustand der Anordnung das oder die Hydrogel-Haftkissen (20, 22, 40, 42, 44, 46, 50, 54, 58) eine Anhaftung des Senders (12, 32) und/oder des Empfängers (14, 34) an der Oberfläche des Gewebes vermittelt oder vermitteln und für einen oder mehrere für die Pulsoxymetrie verwendeten Anteile eines Wellenlängenbereichs des durch das Gewebe gestrahlten Lichts transparent ist oder sind.

2. Anordnung nach Anspruch 1, **dadurch gekennzeichnet, dass** ein Pulsoxymetrie-Monitor umfasst ist, der dazu ausgebildet und mit dem Sensor (10, 30) derart verbunden ist, dass der Pulsoxymetrie-Monitor die Lichtquelle oder Lichtquellen steuert und Signale des Empfängers (14, 34) empfängt und auswertet.

3. Anordnung nach Anspruch 1 oder 2, **dadurch gekennzeichnet, dass** das oder die Hydrogel-Haftkissen (20, 22, 40, 42, 44, 46, 50, 54, 58) hautfreundlich und biokompatibel ist oder sind.

4. Anordnung nach einem der Ansprüche 1 bis 3, **dadurch gekennzeichnet, dass** das oder die Hydrogel-Haftkissen (20, 22, 40, 42, 44, 46, 50, 54, 58) rückstandslos von einer Hautoberfläche entfernbar ist oder sind.

5. Anordnung nach einem der Ansprüche 1 bis 4, **dadurch gekennzeichnet, dass** das oder die Hydrogel-Haftkissen (20, 22, 40, 42, 44, 46, 50, 54, 58) frei von Lufteinschlüssen oder anderen Einschlüssen ist oder sind.

6. Anordnung nach einem der Ansprüche 1 bis 5, **dadurch gekennzeichnet, dass** das oder die Hydrogel-Haftkissen (20, 22, 40, 42, 44, 46, 50, 54, 58) einen Wassergehalt von mehr als 50%, insbesondere von mehr als 80%, aufweist oder aufweisen.

7. Anordnung nach einem der Ansprüche 1 bis 6, **dadurch gekennzeichnet, dass** das oder die Hydrogel-Haftkissen (20, 22, 40, 42, 44, 46, 50, 54, 58) einen stabilisierenden Rand (52, 56, 60), insbesondere aus einem Hydrocolloid, aufweist oder aufweisen.

8. Anordnung nach Anspruch 7, **dadurch gekennzeichnet, dass** der stabilisierende Rand (52, 56, 60) eine Breite von wenigstens 1 mm aufweist.

9. Anordnung nach einem der Ansprüche 1 bis 8, **dadurch gekennzeichnet, dass** der Sensor (10, 30) als Y-Sensor oder in einer U-Konfiguration ausgebildet ist.

10. Verfahren zur Pulsoxymetrie, bei dem aus einem Sender (12, 32) mit einer oder mehreren Lichtquellen Licht durch ein mit Blut versorgtes Gewebe gestrahlt wird und von einem Empfänger (14, 34) aufgefangen wird, wobei aus einer Abschwächung des Lichts bei einer oder mehreren Wellenlängen auf eine Sättigung des in dem Gewebe enthaltenen Blutes mit Sauerstoff ermittelt wird, **dadurch gekennzeichnet, dass** der Sender (12, 32) und/oder der Empfänger (14, 34) mittels eines oder mehrerer beidseitig adhäsiver flächiger Hydrogel-Haftkissen (20, 22, 40, 42, 44, 46, 50, 54, 58) auf einer Oberfläche des Gewebes befestigt wird oder werden, wobei das Licht aus dem Sender (12, 32) vor Eintritt in das Gewebe und/oder nach Austritt aus dem Gewebe ein oder mehrere Hydrogel-Haftkissen (20, 22, 40, 42, 44, 46, 50, 54, 58) durchstrahlt.

11. Verfahren nach Anspruch 10, **dadurch gekennzeichnet, dass** das oder die Hydrogel-Haftkissen (20, 22, 40, 42, 44, 46, 50, 54, 58) zwischen einer Hautoberfäche oder einem Fingernagel (4) einerseits und dem Sender (12, 32) und/oder Empfänger (14, 34) andererseits im Wesentlichen bündig angeordnet wird oder werden.

12. Verfahren nach Anspruch 8 oder 9, **dadurch gekennzeichnet, dass** das oder die Hydrogel-Haftkissen (20, 22, 40, 42, 44, 46, 50, 54, 58) und der Sender (12, 32) und der Empfänger (14, 34) am zu messenden Gewebe, insbesondere einem Finger (2), mit einem, insbesondere einseitig haftenden, Verbandsmaterial (24) umwickelt werden.

13. Verwendung von einem oder mehreren beidseitig adhäsiven flächigen und für Licht transparenten Hydrogel-Haftkissen (20, 22, 40, 42, 44, 46, 50, 54, 58) zur Befestigung eines Senders (12, 32) und/oder eines Empfängers (14, 34) eines Pulsoxymetrie-Sensors (10, 30) auf einem mit Pulsoxymetrie zu überwachenden Gewebes in einem Lichtpfad des Sensors.

Fig. 1

Fig. 2

Fig. 3

a)

51

50

52

b)

44

c)

46

d)

55

56

54

55'

e)

57

60

58

57'

Europäisches
Patentamt

European
Patent Office

Office européen
des brevets

# EUROPÄISCHER RECHERCHENBERICHT

Nummer der Anmeldung

EP 17 16 1538

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2010/324390 A1 (MCLAUGHLIN JAMES ANDREW [GB] ET AL) 23. Dezember 2010 (2010-12-23) <br> * Absätze [0001], [0024], [0046] - [0052] * <br> * Abbildungen 1, 3 * <br> ----- | 1-8, 10-13 | INV. A61B5/00 |
| X | US 2009/171177 A1 (HANNULA DON L [US] ET AL) 2. Juli 2009 (2009-07-02) <br> * Absätze [0027], [0028], [0034] - [0039], [0046], [0047], [0053] * <br> * Abbildungen 1A, 1B, 2, 5A, 7 * <br> ----- | 1-3, 9-11,13 | |
| X | US 2014/194708 A1 (HO HARVEY [US] ET AL) 10. Juli 2014 (2014-07-10) <br> * Absätze [0023] - [0025], [0029], [0031], [0032] * <br> * Abbildungen 1A-1C, 2, 3 * <br> ----- | 1-4,10, 13 | |
| A | US 2008/288026 A1 (CROSS BRETT [US] ET AL) 20. November 2008 (2008-11-20) <br> * Absatz [0048] * <br> * Abbildungen 1,2 * <br> ----- | 7,8 | RECHERCHIERTE SACHGEBIETE (IPC) <br><br> A61B |

Der vorliegende Recherchenbericht wurde für alle Patentansprüche erstellt

| Recherchenort | Abschlußdatum der Recherche | Prüfer |
|---|---|---|
| Den Haag | 11. September 2017 | Trattner, Barbara |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
   ..............................................................................
& : Mitglied der gleichen Patentfamilie, übereinstimmendes
   Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 17 16 1538

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-09-2017

| Im Recherchenbericht angeführtes Patentdokument | Datum der Veröffentlichung | Mitglied(er) der Patentfamilie | Datum der Veröffentlichung |
|---|---|---|---|
| US 2010324390 A1 | 23-12-2010 | AT 527935 T<br>EP 2214551 A1<br>US 2010324390 A1<br>WO 2009056859 A1 | 15-10-2011<br>11-08-2010<br>23-12-2010<br>07-05-2009 |
| US 2009171177 A1 | 02-07-2009 | KEINE | |
| US 2014194708 A1 | 10-07-2014 | EP 3047329 A1<br>US 2014194708 A1<br>US 2014194710 A1<br>WO 2015041717 A1 | 27-07-2016<br>10-07-2014<br>10-07-2014<br>26-03-2015 |
| US 2008288026 A1 | 20-11-2008 | CN 101321494 A<br>EP 1956973 A1<br>JP 2009517160 A<br>US 2008288026 A1<br>WO 2007063436 A1 | 10-12-2008<br>20-08-2008<br>30-04-2009<br>20-11-2008<br>07-06-2007 |

EPO FORM P0461

Für nähere Einzelheiten zu diesem Anhang : siehe Amtsblatt des Europäischen Patentamts, Nr.12/82